# EUROPEAN PATENT APPLICATION

(11) **EP 2 832 306 A1**
(43) Date of publication of application: **04.02.2015**
(21) Application number: 14179046.9
(22) Date of filing: 30.07.2014
(51) Int. Cl.: A61B 17/34

(54) **Instrument or hand access surgical site seal cap**

(30) Priority: 31.07.2013 US 201361860580 P; 22.07.2014 US 201414337430
(71) Applicant: Covidien LP, Mansfield, MA 02048 (US)
(72) Inventor: Furnish, Greg, Louisville, Kentucky 40206 (US)
(74) Representative: Soames, Candida Jane

(57) **Abstract**

A surgical access device comprising a foam structure defining a plurality of generally triangular wedges circumferentially arranged about a longitudinal axis so as to seal the surgical incision, the plurality of generally triangular wedges defining, adjacent their respective apices, a central opening for sealed reception of a surgical object, and a second opening defined between a pair of adjacent wedges, the second opening configured to receive and seal with a surgical instrument inserted into the second opening.

## Description

### CROSS-REFERENCE TO RELATED APPLICATIONS

This application claims the benefit of and priority to U.S. Provisional Patent Application No. 61/860,580, filed July 31, 2013, the entire disclosure of which is incorporated by reference herein.

### TECHNICAL FIELD

The present application is directed to various embodiments of instrument or hand access surgical site seal caps that improve a surgical seal while providing greater flexibility for manipulation of instruments and tactile feel to the surgeon and thus enhanced accuracy when either a hand or an instrument is disposed through the seal cap.

### SUMMARY

The present disclosure relates to instrument or hand access surgical site seal caps that provide various configurations to effect improvements in the surgical seal while providing the above-mentioned greater flexibility for manipulation of instruments and tactile feel to the surgeon and thus enhanced accuracy when either a hand or an instrument is disposed through the seal cap.

In one exemplary embodiment, the present disclosure relates to an instrument or hand access surgical site seal cap that includes a port assembly that includes a proximal seal retaining portion and a distal seal retaining portion. First and second composite seals are engaged within the port assembly and each have a J-shaped configuration. The first composite seal and the second composite seal interface each other such that the J-shaped configuration of the first and second composite seals are each vertically disposed and interface each other to form a surgical site access location that effects a seal when an instrument or a hand is, or an instrument and a hand are, disposed therethrough.

In another exemplary embodiment, an instrument or hand access surgical site seal cap includes a port assembly that includes a proximal seal retaining portion and a distal seal retaining portion. The port assembly includes a plurality of layers of a flexible material having various patterns of access ports for hand access and large cross-sectional area instrument access, wherein the patterns of the access ports include football, oval, or cone-shaped access patterns.

In another exemplary embodiment, the present disclosure relates to an instrument or hand access surgical site seal cap that includes a flexible bag having a proximal end that is open and that is connected to a proximal circular interlock rim and has a distal end that is open and that is connected to a distal snap-on cap. A lanyard is positioned on an external surface of the flexible bag, the lanyard looping around the external surface of the bag. The lanyard includes a sliding, releasable lock that is positioned on the lanyard and is moved along a portion of the length of the lanyard until an aperture having a desired diameter is formed in the flexible bag by the lanyard. The desired aperture has a cross-sectional area corresponding to an approximate cross-sectional area of an instrument or a hand that is intended to be inserted through, and sealed by, the site seal cap.

In yet another exemplary embodiment, an instrument or hand access surgical site seal cap includes a flexible bag. A proximally positioned interlock rim includes two rigid members, the first rigid member defining a circular twist rim. A snap on cap is positioned distally from the interlock rim. A lanyard is located at a proximal end of the flexible bag. The proximal end extends proximally from the interlock rim.

In yet another exemplary embodiment, an instrument or hand access surgical site seal cap includes a surgical site port structural member and a plurality of flexible interlocking wedges. The wedges are positioned adjacent to one another spanning a 360° circle within the surgical site port structural member. Each wedge defines first and second vertical lateral surfaces that intersect at an apex. Each wedge tapers from a height at a base of the wedge to a height at the apex such that the first lateral surface and the second lateral surface form an arched profile and each wedge defines a generally contoured pyramidal configuration.

In a further exemplary embodiment, an instrument or hand access surgical site seal cap includes a surgical site port structural member and a plurality of flexible triangular wedges alternately spaced apart with respect to one another within the surgical site port structural member. Each triangular wedge defines a respective apex that converges at a vertical centerline of the surgical site port structural member. Each wedge defines first and second vertical lateral surfaces that intersect at the apex. The wedges define a first lateral surface and a second lateral surface that intersect at the respective apex. Each wedge tapers from a height at a base of the wedge to a height at the apex such that first lateral surface and second lateral surface form an arched profile and each wedge defines a generally contoured pyramidal configuration.

In a still further exemplary embodiment, an instrument or hand access surgical site seal cap includes a retaining housing encasing a flexible material that is divided into a plurality of segments such that a plurality of vertical access pierce slits are formed at the interfaces between each segment and an adjacent segment. Each segment includes a respective arcuate spring support formed within the flexible material of the respective segment.

In another exemplary embodiment, an instrument or hand access surgical site seal cap includes a housing and a soft material mounted in the housing. The soft material defines pierce slits to form a plurality of soft material segments, wherein the pierce slits have a curled wave-like vertical profile to form interlocking ribs to improve sealing capability of the seal.

The present invention, in accordance with various embodiments thereof, may also relate to a surgical access device comprising a foam structure defining a plurality of generally triangular wedges circumferentially arranged about a longitudinal axis so as to seal the surgical incision, the plurality of generally triangular wedges defining, adjacent their respective apices, a central opening for sealed reception of a surgical object, and a second opening defined between a pair of adjacent wedges, the second opening configured to receive and seal with a surgical instrument inserted into the second opening.

In various embodiments, each wedge has a first vertical surface on a first side and a second vertical surface on a second side. The first vertical surface of a first wedge may abut a second vertical surface of a second wedge. The first vertical surface of the first wedge and the second vertical surface of the second wedge may form a slit between the first and second wedges, and the slit formed between the first vertical surface of the first wedge and the second vertical surface of the second wedge may extend fully between an upper surface and a lower surface of the surgical access device. The second opening may be in communication with at least a part of the slit.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure:
FIG. 1 is a perspective cross-sectional view of an instrument or hand access surgical site seal cap according to one exemplary embodiment of the present disclosure that includes a generally cylindrical site access port assembly having composite interfacing J-shaped layers that effect a seal;
FIG. 2 is a perspective view of the instrument or hand access surgical site seal cap of FIG. 1;
FIG. 3 is a perspective cross-sectional view of an instrument or hand access surgical site seal cap according to another exemplary embodiment of the present disclosure that includes several layers of a flexible material having various patterns of access ports for hand access and large cross-sectional area instrument access;
FIG. 4 is a perspective plan view of the entire instrument or hand access surgical site seal cap of FIG. 3;
FIG. 5 is a an enlarged plan view of the instrument or hand access surgical site seal cap of FIGS. 3 and 4 further including access ports for small cross-sectional area instrument access;
FIG. 6A is a plan view of a flexible material layer included within the instrument or hand access surgical site seal cap of FIGS. 3, 4 and 5 and configured to enable hand access;
FIG. 6B is a plan view of a flexible material layer included within the instrument or hand access surgical site seal cap of FIGS. 3, 4 and 5 that is configured with small access ports to enable access for small cross-sectional area instruments and which underlies the flexible material layer for hand access of FIG. 6A;
FIG. 6C is a plan view of a flexible material layer included within the instrument or hand access surgical site seal cap of FIGS. 3, 4 and 5 that is configured with small access ports to enable access for small cross-sectional area instruments and an alternative pattern which underlies the flexible material layer for hand access of FIG. 6A;
FIG. 7 is an exploded view of several of the layers of flexible material of the instrument or hand access surgical site seal cap of FIGS. 3, 4 and 5 and configured to enable small cross-sectional area instruments, large cross-sectional area instruments and hand access according to one exemplary embodiment of the present disclosure;
FIG. 8 is a plan view of the several layers of flexible material of FIG. 7;
FIG. 9 is an exploded view of several layers of flexible material of the instrument or hand access surgical site seal cap of FIGS. 3, 4 and 5 and configured to enable small cross-sectional area instruments and hand access according to one exemplary embodiment of the present disclosure;
FIG. 10 is a perspective view of an instrument or hand access surgical site seal cap in an extended position showing the interfacing configuration of a snap-on cap to a wound retractor according to one exemplary embodiment of the present disclosure;
FIG. 11 is a perspective view of the instrument or hand access surgical site seal cap of FIG. 10 in a collapsed configuration prior to sealing the shaft of an instrument;
FIG. 12 is a perspective view of the instrument or hand access surgical site seal cap of FIG. 11 in an extended configuration subsequent to sealing the shaft of an instrument;
FIG. 13 is a perspective view of an instrument or hand access surgical site seal cap according to one exemplary embodiment of the present disclosure which includes two sealing locations;
FIG. 14 is an exploded perspective view of the instrument or hand access surgical site seal cap of FIG. 13;
FIG. 15 is a perspective view of the instrument or hand access surgical site seal cap of FIGS. 13 and 14 in an extended configuration prior to sealing the shaft of an instrument;
FIG. 16 is a plan view of an instrument or hand access surgical site seal cap that includes a plurality of flexible interlocking wedges adjacent to one another within a cylindrical surgical site port according to one exemplary embodiment of the present disclosure;
FIG. 17 is a perspective view of the instrument or hand access surgical site seal cap of FIG. 16 illustrating one of the flexible interlocking wedges in a raised position above the cylindrical surgical site port;
FIG. 18 is a plan view of the instrument or hand access surgical site seal cap of FIGS. 16 and 17 illustrating positioning of a small cross-sectional area instrument and a large cross-sectional area instrument within the surgical site seal cap;
FIG. 19 is a perspective view of an instrument or hand access surgical site seal cap that includes a plurality of flexible wedges alternately spaced apart with respect to one another within a cylindrical surgical site port according to one exemplary embodiment of the present disclosure;
FIG. 20 is a perspective cross-sectional view of the instrument or hand access surgical site seal cap of FIG. 19 taken through two of the flexible wedges;
FIG. 21 is a perspective frontal view of one of the flexible wedges of FIGS. 19 and 20 showing a series of ribs extending along lateral surfaces of the flexible wedge;
FIG. 22 is a perspective rear view of the flexible wedge of FIG. 21 showing a hollow internal volume within the flexible wedge;
FIG. 23 is a perspective view of an instrument or hand access surgical site seal cap having a series of flexible arcuate spring supports positioned within a soft rubber-like material such that the supports limit horizontal movement of the material but allow vertical movement according to one exemplary embodiment of the present disclosure;
FIG. 24 is an elevation cross-sectional view of the instrument or hand access surgical site seal cap of FIG. 23 showing the flexible arcuate spring supports positioned within the soft rubber-like material;
FIG. 25 is a plan view of the instrument or hand access surgical site seal cap of FIGS. 23 and 24 showing the central intersection of pierce slits in the rubber-like material that are positioned between the flexible arcuate spring supports;
FIG. 26 is a plan view of the instrument or hand access surgical site seal cap of FIG. 25 illustrating the deformation of the flexible arcuate spring supports when a hand or an instrument is disposed symmetrically through the central intersection of the pierce slits;
FIG. 27 is a plan view of the instrument or hand access surgical site seal cap of FIG. 25 illustrating the deformation of the flexible arcuate spring supports when a hand or an instrument is disposed asymmetrically through the central intersection of the pierce slits and an instrument having a small cross-sectional area is disposed asymmetrically through one of the pierce slits in the rubber-like material; and
FIG. 28 is a cross-sectional view of an alternate exemplary embodiment of the instrument or hand access surgical site seal cap of FIGS. 23-27 wherein the pierce slits have a curled wave-like vertical profile to form interlocking ribs to improve the seal capability.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is further from the user. In the following description, well-known functions or constructions are not described in detail to avoid obscuring the present disclosure in unnecessary detail.

The present disclosure relates to various embodiments of instrument or hand access surgical site seal caps that enable access to a surgical site with either a surgeon's hand and/or one or more instruments.

As defined herein, a surgical site includes an incision in a patient or a natural orifice through which a surgical procedure may be performed.

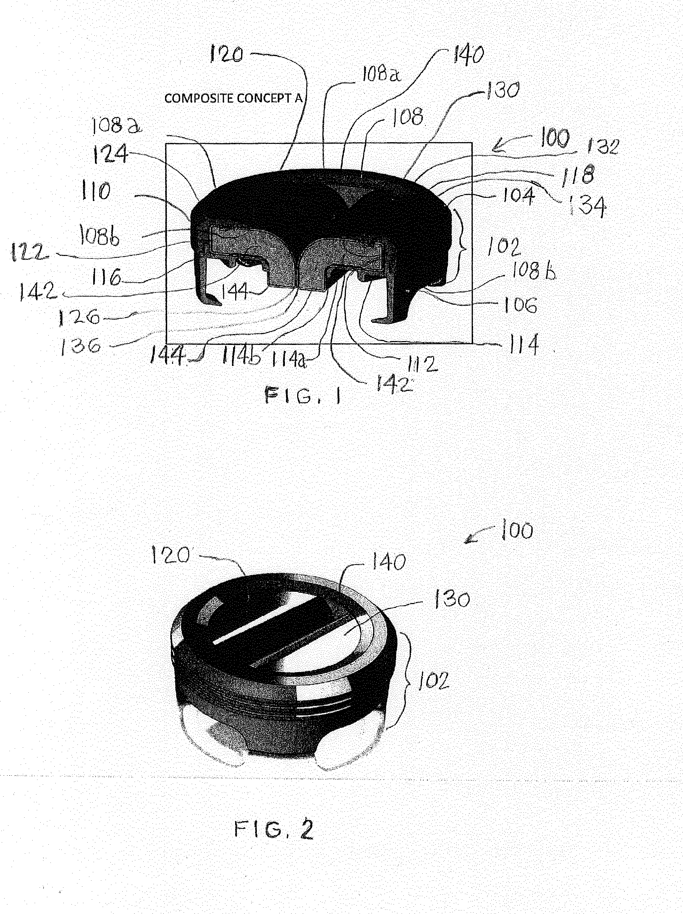

FIGS. 1 and 2 illustrate an instrument or hand access surgical site seal cap 100 according to one exemplary embodiment of the present disclosure. Surgical site seal cap 100 includes a generally cylindrical site access port assembly 102 that includes an upper or proximal seal retaining portion 104 and a lower or distal seal retaining portion 106. Alternatively, one skilled in the art will readily appreciate that the site access port assembly 102 may have a polygonal, oval or rectangular (form of polygonal) configuration according to the intended usage The upper seal retaining portion 104 has a J-shaped cross-section defining an inwardly projecting rim portion 108 having an upper surface 108a and a lower surface 108b. Upper seal retaining portion 104 further defines a peripheral wall 110 that includes an inner vertical surface 112.

Lower or distal seal retaining portion 106 has a generally irregular J-shaped cross-section that includes an inwardly projecting rim portion 114 having an upper or proximal surface 114a and a lower or distal surface 114b. Lower or distal seal retaining portion 106 defines a peripheral wall 116.

A vertically projecting rim portion 118 is spaced circumferentially around the upper surface 114a of, and engages with, a slot defined in the peripheral wall 110 of the upper or proximal seal retaining portion 104.

One end 122 of a first composite seal 120 is engaged within the space formed by the lower surface 108b of the upper seal retaining portion 104 and the upper surface 114a of the lower seal retaining portion 106. A projection 124 extending vertically downward from the lower surface 108b penetrates into the first composite seal 120 to enhance stability.

Similarly, one end 132 of a second composite seal 130 is engaged within the space formed by the lower surface 108b of the upper seal retaining portion 104 and the upper surface 114a of the lower seal retaining portion 106. In a similar manner, a projection 134 extending vertically downward from the lower surface 108b penetrates into the second composite seal 130 to enhance stability.

The first composite seal 120 and the second composite seal 130 each have a J-shaped cross-section and are disposed such that second end 126 of the first seal portion 120 and second end 136 of the second seal portion 130 are each vertically disposed and interface each other to form a surgical site access location 140 that is configured as a straight line and thus effects a seal.

Each seal 120, 130 includes a closed cell foam core 142 defining the J-shaped cross-section. The core 142 of each seal 120, 130 is encased by a top and bottom dipped liquefied medical grade material elastomeric sheeting 144 such as silicone membrane used for breast implants such as a medical grade plastic including, but not limited to, polyisoprene, urethane, silicone, or any other material suitable for the intended purpose of facilitating off-axis movement of an inserted surgical instrument or clinician's hand, e.g., a flowable or sufficiently compliable material, such as an open-cell polyurethane foam, a thermoplastic elastomer (TPE), or a gel.

The aforementioned components of site access port assembly 102 may be rigid members as described above or alternatively made from a disposable, compressible, and/or flexible type material, for example, but not limited to, a suitable foam or gel material having sufficient compliance to form a seal about one or more surgical objects, shown generally as surgical object, and also establish a sealing relation with the tissue. The foam is preferably sufficiently compliant to accommodate off axis motion of the surgical object. In one embodiment, the foam may be at least partially constituted of polyisoprene, urethane, or silicone, or the like. Alternatively, site access port assembly 102 may be formed of a biocompatible gel material. Suitable portal members are disclosed in commonly assigned U.S. patent application Ser. No. 12/244,024, filed Oct. 2, 2008, now abandoned, published as U.S. Patent Application Publication No. 2009/0093752 A1, the entire contents of which is hereby incorporated by reference herein. A hand or instrument is able to access a surgical site by insertion through the surgical site access location 140.

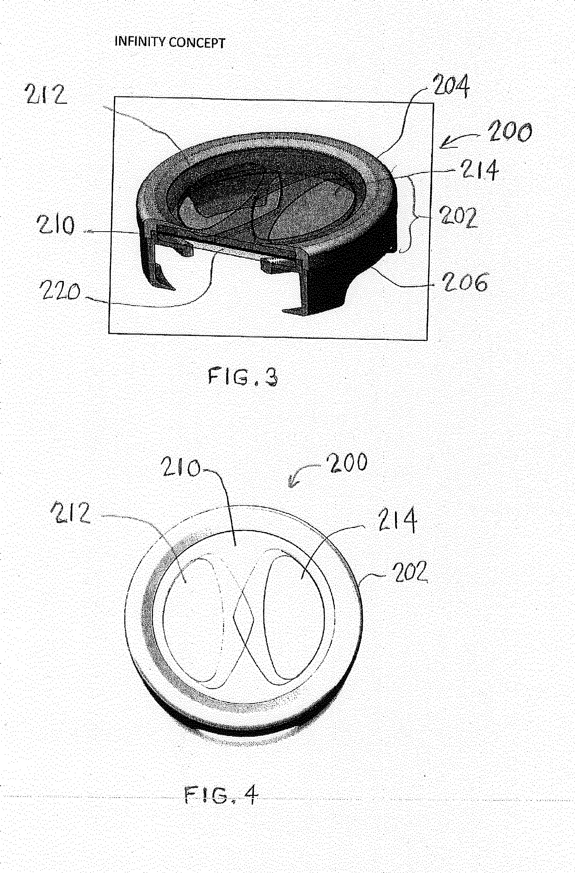

Turning now to FIGS. 3-9, there is disclosed an instrument or hand access surgical site seal cap 200 according to another exemplary embodiment of the present disclosure that is generally similar to the instrument or hand access surgical site seal cap 100 described above.

In a similar manner, surgical site seal cap 200 includes a generally cylindrical site access port assembly 202 that includes an upper or proximal seal retaining portion 204 and a lower or distal seal retaining portion 206. Due to the similarities, the site access port assembly 202 is not described in more detail.

However, in place of the two interfacing J-shaped composite seals 120 and 130, the surgical site seal cap 200 includes several layers of a flexible material having various patterns of access ports for hand access and large cross-sectional area instrument access.

More particularly, in one exemplary embodiment, as best illustrated in FIG. 3, surgical site seal cap 200 includes an upper layer 210 of flexible material such as a medical-grade plastic or a foam core encased by a top and bottom dipped liquefied medical grade material elastomeric sheeting, such as polyisoprene, urethane, silicone, or any other material suitable for the intended purpose of facilitating off-axis movement of an inserted surgical instrument or clinician's hand, e.g., a flowable or sufficiently compliable material, such as an open-cell polyurethane foam, a thermoplastic elastomer (TPE), or a gel.

The layer 210 defines therein a first port 212 that is generally football or oval shaped and a second port 214 that is defined therein and is spaced apart from the first port 212. The second port 214 has a cone-shaped configuration with an aperture area that is greater than the aperture area of the first port 212.

The difference in aperture areas enables instruments of different cross-sectional areas or a surgeon's hand to be inserted through the ports 212 and 214.

Although shown in a football or oval shape configuration, one skilled in the art will readily appreciate that the ports 212 and 214 can be made in any shape depending on the application.

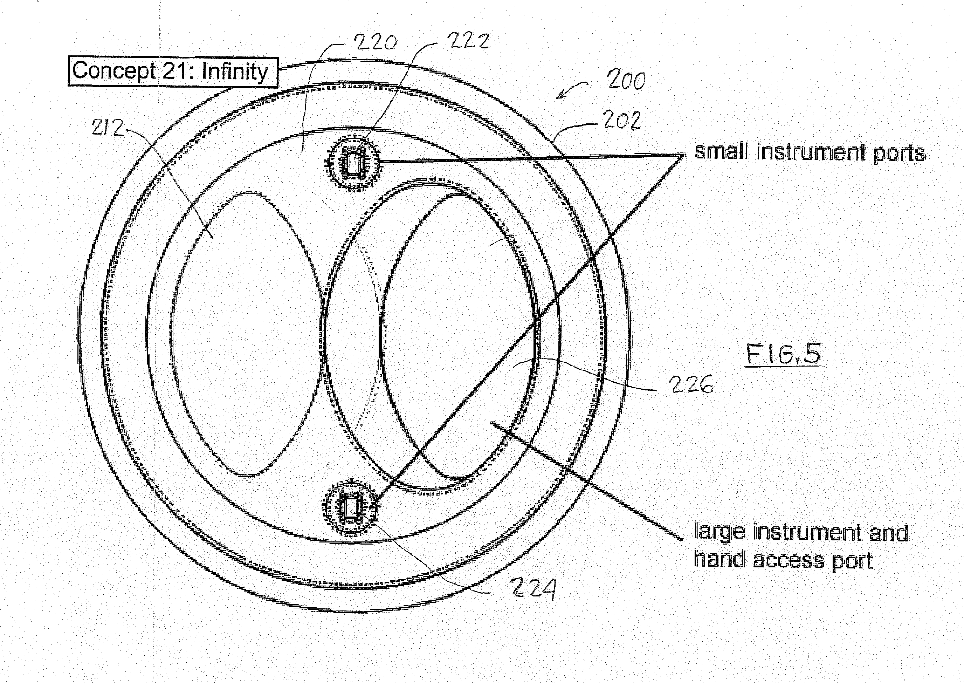

As illustrated in FIG. 5, an alternate embodiment of layer 210, designated as layer 220, further defines a first small instrument port 222 and a second small instrument port 224. The layer 220 includes an alternatively football or oval shaped second port 226 in contrast to second port 214.

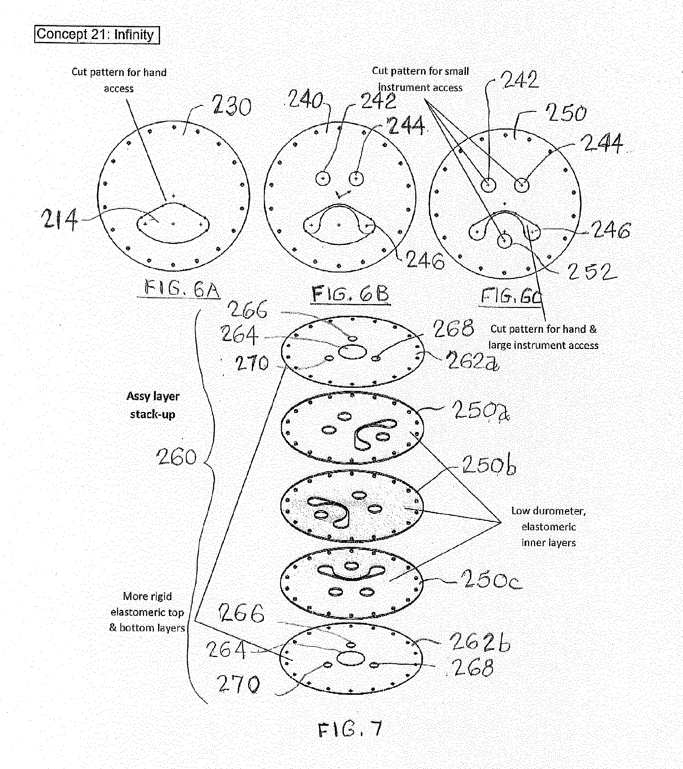

FIGS. 6A, 6B, 6C illustrate alternate patterns of ports defined in various flexible material layers of the instrument or hand access surgical site seal cap 200. More particularly, FIG. 6A illustrates a flexible material layer 230 included within the instrument or hand access surgical site seal cap 200 and includes the cone-shaped port 214 defined therein which is configured to enable hand access.

FIG. 6B illustrates another flexible material layer 240 included within the instrument or hand access surgical site seal cap 200 that is configured with small access ports 242 and 244 to enable access for small cross-sectional area instruments and which underlies the flexible material layer 230 for hand access of FIG. 6A. The flexible material layer 240 further includes a curved bar-bell shaped port 246 defined therein that is disposed adjacent the small access ports 242 and 244 and enables either hand or large instrument access.

FIG. 6C illustrates yet another flexible material layer 250 that includes the small access ports 242 and 244 and the curved bar-bell shaped port 246. The flexible material layer 250 further includes a third small instrument access port 252 defined in the flexible material layer 250 such that the curved bar-bell shaped port 246 is disposed between the small ports 242, 244 and the third small port 252 and in closer proximity to the third small instrument access port. The flexible material layer 250 may be disposed to underlie the flexible material layers 230 and 240.

FIG. 7 is an exploded view of a stack 260 of several of the layers of flexible material of the instrument or hand access surgical site seal cap 200 and which are configured to enable small cross-sectional area instruments, large cross-sectional area instruments and hand access according to one exemplary embodiment of the present disclosure. More particularly, five flexible material layers 262a, 250a, 250b, 250c and 262b are illustrated disposed in a stacked configuration in the stack 260 from top to bottom, respectively. The first flexible material layer 262a and the fifth flexible material layer 262b are substantially similar to each other and each defines a central aperture 264 surrounded by three small instrument apertures 266, 268 and 270 spaced apart 120° with respect to each other and each one equidistant from the center of the central aperture 264.

The second, third and fourth flexible material layers 250a, 250b and 250c are substantially similar to the flexible material layer 250 of FIG. 6C and are oriented such that the access ports in each layer are shifted 120° with respect to the access ports in the adjacent stacked layer. It is further contemplated that more or less than the five flexible material layers can be employed depending upon the specific application contemplated by the user. In some embodiments, the outermost layers are of a different thickness than the innermost layers, for example thicker.

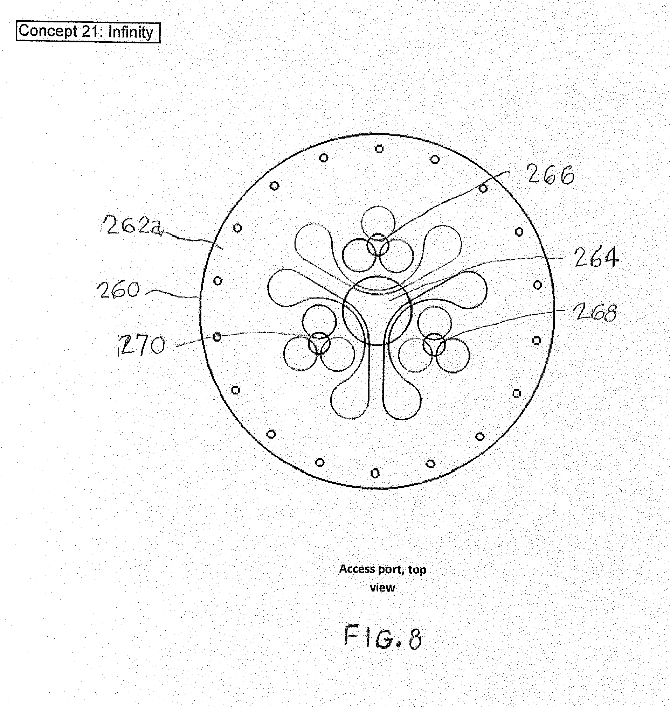

FIG. 8 is a plan view of the several flexible material layers 262a, 250a, 250b, 250c and 262b as they appear stacked in the order described above with respect to FIG. 7 with the first layer 262a being on top.

The access ports described above such as the small access ports 242 and 244 and the curved bar-bell shaped port 246. or the first access port 212 or the second access port 214, etc., could be any shape including round, oval, slits, etc. depending on the intended usage.

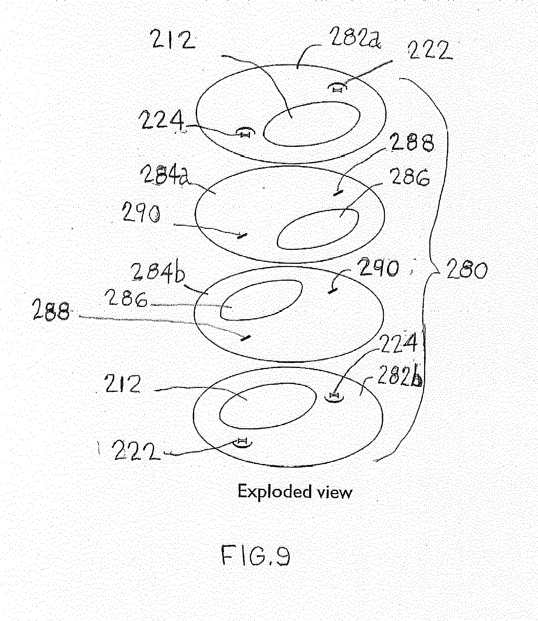

FIG. 9 is an exploded view of an alternate embodiment of a stack 280 of several layers of flexible material of the instrument or hand access surgical site seal cap 200 of FIGS. 3, 4 and 5 and which are configured to enable small cross-sectional area instruments and hand access.

More particularly, stack 280 includes a first flexible material layer 282a and a fourth flexible material layer 282b that are substantially the same as to the flexible material layer 220 described above with respect to FIG. 5. A second flexible material layer 284a and a third flexible material layer 284b which are similar to each other each includes a football or oval shaped access port 286 defined therein and a first narrow slit access port 288 and a second narrow slit access port 290 that are disposed adjacent to the oval shaped access port 286 on either end and to one side of the oval shaped access port 286.

The first flexible material layer 282a and the second flexible material layer 284a are disposed such that the first small instrument port 222 and second small instrument port 224 and the football or oval shaped second port 212 of the first layer 282a generally coincide with the football or oval shaped access port 286 and first narrow slit access port 288 and second narrow slit access port 290 of the second flexible material layer 284a.

Similarly, the third flexible material layer 284b and the fourth flexible material layer 282b are disposed such that the football or oval shaped access port 286 and first narrow slit access port 288 and second narrow slit access port 290 of the third flexible material layer 284b generally coincide with the first small instrument port 222 and second small instrument port 224 and the football or oval shaped second port 212 of the fourth layer 282b.

However, the third layer 284b and the fourth layer 282b are disposed such that their respective access ports are oriented 180° with respect to the respective access ports of the first layer 282a and second layer 284a.

The flexible materials included within the surgical site seal cap 200 may be made from the flexible materials described above.

Again, the football or oval shaped access port 286, first narrow slit access port 288, second narrow slit access port 290, first small instrument port 222, second small instrument port 224, football or oval shaped second port 226, first narrow slit access port 288 and second narrow slit access port 290 could be any shape including round, oval, slits, etc. depending on the intended usage.

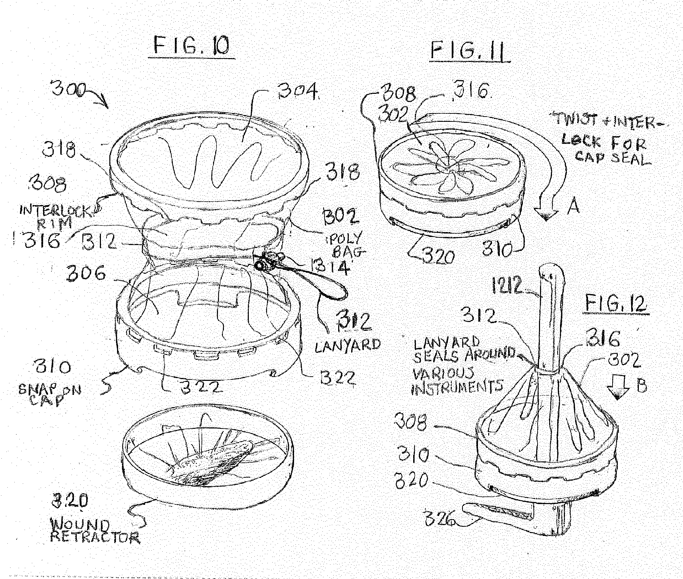

FIG. 10 illustrates an instrument or hand access surgical site seal cap 300 in an extended position showing the interfacing configuration of a snap-on cap to a wound retractor according to one exemplary embodiment of the present disclosure. More particularly, instrument or hand access surgical site seal cap 300 includes a flexible bag 302 having a proximal or first end 304 that is open and that is connected to a proximal circular interlock rim 308 and having a distal or second end 306 that is open and that is connected to a distal circular snap-on cap 310.

A lanyard 312 is positioned on the external surface of the flexible bag 302 and loops around the external surface of the bag 302. A sliding, releasable lock 314 is positioned on the lanyard 312 and is moved along at least a portion of the length of the lanyard 312 until an aperture 316 having a desired diameter is formed in the flexible bag 302 by the lanyard 312. The desired aperture 316 has a cross-sectional area corresponding to the approximate cross-sectional area of an instrument or a hand that is intended to be inserted through, and sealed by, the site seal cap 300.

A wound retractor 320 is positioned distally of the snap on cap 310. The snap on cap 310 is snapped or locked on to the wound retractor 320. The interlock rim 308 includes a plurality of distally protruding projections 318 that are configured to engage with a plurality of proximally positioned reception grooves 322 defined in the snap on cap 310.

When the interlock rim 308, the snap on cap 310 and the wound retractor 320 are sequentially engaged to one another into a collapsed configuration by twisting interlock rim 308 in the direction of arrow with respect to the snap on cap 310 as shown in FIG. 11, the flexible bag 302, due to the excess of material now available, forms an iris configuration around the aperture 316.

Referring now to FIG. 12, the aperture 316 of the flexible bag 302 is positioned around the shaft 324 of an instrument 326 and the lanyard 312 is further tightened around the shaft 324 by moving the lock 314 to provide a seal around the shaft 324. The surgical site seal cap 300 is then moved vertically downward in the direction of arrow B along the shaft 324, and the shaft 324 may be moved vertically upward in the opposite direction, to tighten the seal at the aperture 316 and to form the flexible bag 302 into a cone shaped extended configuration extending vertically upward (opposite to arrow B) as shown in FIG. 12 subsequent to sealing of the shaft 324.

Although described as effected by a lanyard, the resulting cinching effect produced by the lanyard 312 can be produced by any conventional known means for pursing or reducing the diameter.

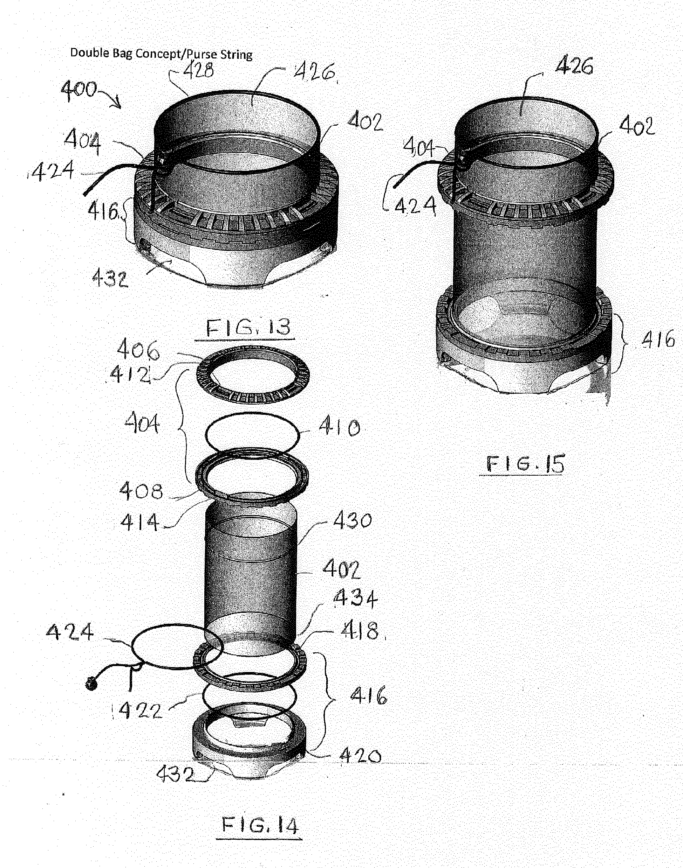

FIGS. 13-15 illustrate an instrument or hand access surgical site seal cap 400 according to one exemplary embodiment of the present disclosure that is similar to the surgical site seal cap 300 described above with respect to FIGS. 10-12 except that surgical site seal cap 400 includes a proximally located lanyard and two sealing locations. More particularly, surgical site seal cap 400 includes a flexible bag 402 and proximally positioned interlock rim 404 that includes two rigid members 406 and 408 and an O-ring seal 410. The first rigid member 406 is a circular twist rim 406 that defines an aperture 412 for receiving a projection 414 extending from the second rigid member 408 that is a circular interlock that is positioned distally from the circular rim 406. The O-ring seal 410 is positioned between the circular rim 406 and the circular interlock 408. The proximally positioned interlock rim 404 engages with a groove 430 disposed in the vicinity of proximal end 428 of, and on an external surface of, the flexible bag 402.

A snap on cap 416 is positioned distally from the interlock rim 404. The snap on cap 416 includes a circular engaging rim 418 that receives the circular interlock 408. The snap on cap 416 further includes a snap on cap base 420 that is positioned distally from the circular engaging rim 418 and an O-ring seal 422. The O-ring seal 422 is positioned between the circular engaging rim 418 and the snap on cap base 420. A lanyard 424 is located at proximal end 428 of the flexible bag 402. The proximal end 428 extends proximally from the interlock rim 404.

If there is no instrument or hand present, the circular twist rim 406 is twisted with respect to the circular interlock 408 to seal wound protector 432 to which the snap on cap 416 attaches. Upon insertion of an instrument (not shown) into the proximal aperture 426, the lanyard 424, which is positioned at proximal aperture 426 defined at proximal end 428, is tightened to form a seal and the circular twist rim 424 is loosened to allow access to the wound protector 432 and surgical cavity (not shown) via distal aperture 434 of the flexible bag 402.

Although described as effected by a lanyard, in a similar manner as described above with respect to lanyard 312, the resulting cinching effect produced by the lanyard 424 can be produced by any conventional known means for pursing or reducing the diameter.

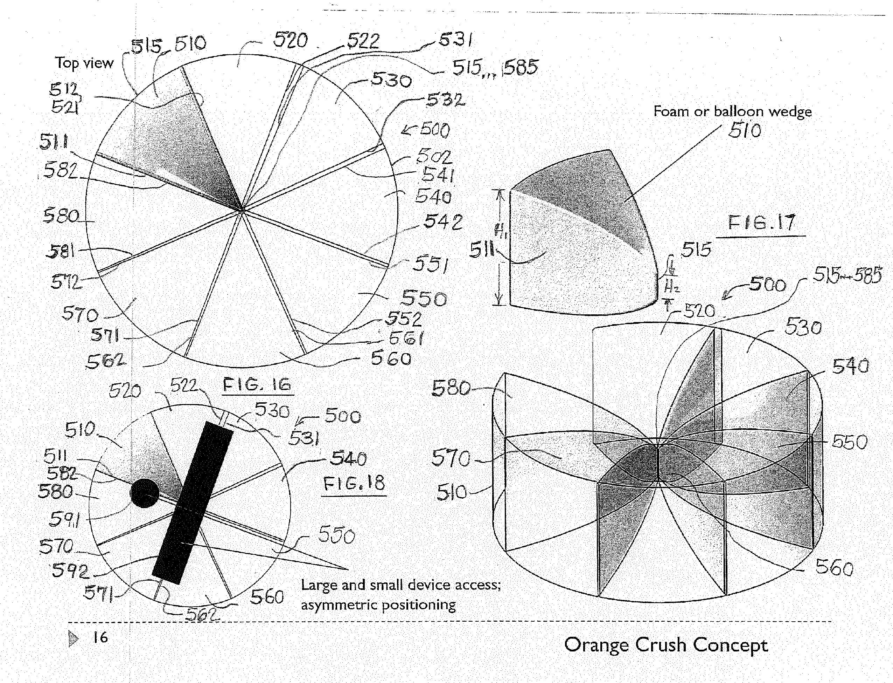

FIG. 16 illustrates an instrument or hand access surgical site seal cap 500 that includes a plurality of flexible interlocking triangular wedges adjacent to one another within a cylindrical surgical site port according to one exemplary embodiment of the present disclosure. More particularly, instrument or hand access surgical site seal cap 500 includes a plurality of flexible triangular interlocking wedges, e.g., first wedge 510, second wedge 520, third wedge 530, fourth wedge 540, fifth wedge 550, sixth wedge 560, seventh wedge 570 and eighth wedge 580.

Although shown with eight wedges, one skilled in the art will readily appreciate more or fewer than eight wedges may be used depending on the application.

As best illustrated in FIG. 17, the wedges 510-580 are positioned adjacent to one another spanning a 360° circle within a cylindrical surgical site port structural member 502. One of the flexible interlocking wedges, i.e., wedge 510, is illustrated in a raised position above the cylindrical surgical site port structural member 502.

Each wedge 510...580 defines a respective apex or tip 515...585, respectively, that all converge at the vertical centerline of the cylindrical surgical site port structural member 502. Each wedge defines first and second vertical lateral surfaces that intersect at the apex. More particularly, first to eighth wedges 510...580 define a first lateral surface 511, 521, 531, 541, 551, 561, 571 and 581 and a second lateral surface 512, 522, 532, 542, 552, 562, 572 and 582 that intersect at the respective apices 515...585.

Each wedge 510...580 tapers from a height H1 at base 515 to a height H2 at the respective apices 515...585 such that first lateral surface 511, 521, 531, 541, 551, 561, 571 and 581 and second lateral surface 512, 522, 532, 542, 552, 562, 572 and 582 form an arched profile and each wedge defines a generally contoured pyramidal configuration.

First lateral surface 511 of wedge 510 is disposed adjacent to second lateral surface 582 of eighth wedge 580 to define a first radial access split to enable access of an instrument or a surgeon's hand therethrough. Similarly, second lateral surface 512 of wedge 510 is disposed adjacent to first lateral surface 521 of second wedge 520 to define a second radial access split.

Those skilled in the art will recognize that, and understand how, third, fourth, fifth, sixth, seventh and eighth radial access splits are defined between the third through eighth wedges 530...580, respectively.

FIG. 18 illustrates the instrument or hand access surgical site seal cap 500 of FIGS. 16 and 17 wherein a small cross-sectional area instrument 591 having a circular cross section is positioned in the radial access split formed between first lateral surface 511 of first wedge 510 and second lateral surface 582 of eighth wedge 580.

In addition, a large cross-sectional area instrument 592 having a rectangular cross-section is positioned generally in the radial access split formed between second lateral surface 522 of second wedge 520 and first lateral surface 531 of third wedge 530 and between second lateral surface 562 of sixth wedge 560 and first lateral surface 571 of seventh wedge 570.

Thus the surgical site seal cap 500 enables either symmetric or asymmetric positioning of a large or a small instrument or a surgeon's hand through the seal cap. The wedges 510...580 may be formed of a medical grade plastic or medical grade foam which may or may not be encased in a silicone material as described above or formed of a medical balloon material, e.g., latex, silicone rubber, Mylar®, polyethylene, polyurethane, a flexible polymeric material, or a composite material, such as a polyethylene and nylon composite, or any other resilient material suitable for the intended purpose of permitting insertion and manipulation, e.g., off-axis manipulation, of a surgical instrument or clinician's hand

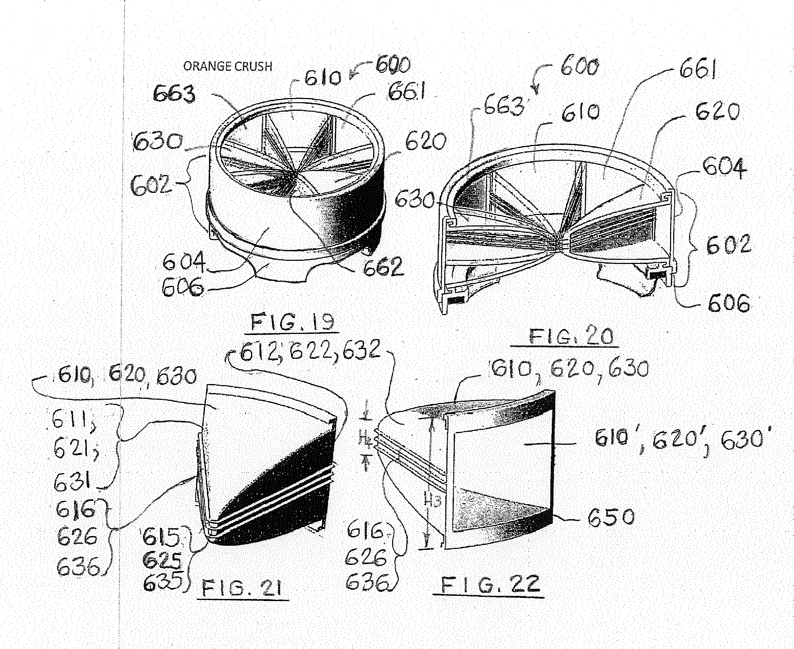

FIGS. 19 and 20 illustrate an instrument or hand access surgical site seal cap 600 that is similar to the surgical site access cap 500 described above with respect to FIGS. 16-18 except that instrument or hand access surgical site seal cap 600 includes a plurality of flexible triangular wedges, e.g., first triangular wedge 610, second triangular wedge 620 and third triangular wedge 630, alternately spaced apart with respect to one another within a cylindrical surgical site port structural member 602 according to one exemplary embodiment of the present disclosure.

The cylindrical surgical site port structural member 602 includes an upper cylindrical portion 604 and a corresponding circular ring base member 606 that engages the lower end of the upper cylindrical portion 604.

Turning now to FIGS. 21-22, each triangular wedge 610, 620, 630 defines a respective apex or tip 615, 625, 635, respectively, that converge at the vertical centerline of the cylindrical surgical site port structural member 602. Each wedge defines first and second vertical lateral surfaces that intersect at the apex. More particularly, first, second and third wedges 610, 620, 630 define a first lateral surface 611, 621, 631, and a second lateral surface 612, 622, 632 that intersect at the respective apices 615, 625, 635.

In a similar manner as with respect to surgical site seal cap 500 described above, each wedge 610, 620, 630 tapers from a height H3 at base 650 to a height H4 at the respective apices 615, 625, 635 such that first lateral surface 611, 621, 631 and second lateral surface 612, 622, 632 form an arched profile and each wedge defines a generally contoured pyramidal configuration.

The wedges 610-630 each include a set of reinforcing ribs 615, 625, 635, respectively, that extend horizontally along the first lateral surfaces 611, 621, 631, extending around the apices 615, 625, 635 and further extending horizontally along the second lateral surfaces 612, 622, 632, respectively.

As illustrated in FIG. 22, the rear portion or base 650 of each wedge 610, 620, 630 defines an aperture or hollow internal volume 610', 620', 630', respectively, such that each wedge 610, 620, 630 is effectively hollow.

Returning to FIGS. 19 and 20, the wedges 610-630 are disposed within the upper cylindrical portion 604 of the cylindrical surgical site port structural member 602 equally spaced apart along the circumference of the upper cylindrical portion 604 to form a first triangular wedge-shaped space 661 between the first wedge 610 and the second wedge 620, a second triangular wedge-shaped space 662 between the second wedge 620 and the third wedge 630, and a third triangular wedge-shaped space 663 between the third wedge 630 and the first wedge 610. In the exemplary embodiment of instrument or hand access surgical site seal cap 600, each wedge 610, 620, 630 forms a 60° angle at the respective apex 615, 625, 635 such that the first, second and third triangular wedge-shaped spaces 661, 662, 663 also form a 60° angle at their respective apices.

Thus, as can be appreciated, instrument or hand access surgical site seal cap 600 enables passage of either small or large cross-sectional area instruments or a surgeon's hand either symmetrically or asymmetrically through the spaces 661, 662, 663 or through the vertical slit defined by the intersection of the apices 615, 625, 635.

Again, the wedges 610, 620, 630 and the respective reinforcing ribs 616, 626, 636 may be formed of a medical grade plastic or medical grade foam which may or may not be encased in a silicone material as described above or formed of a medical balloon material, e.g., latex, silicone rubber, Mylar®, polyethylene, polyurethane, a flexible polymeric material, or a composite material, such as a polyethylene and nylon composite, or any other resilient material suitable for the intended purpose of permitting insertion and manipulation, e.g., off-axis manipulation, of a surgical instrument or clinician's hand.

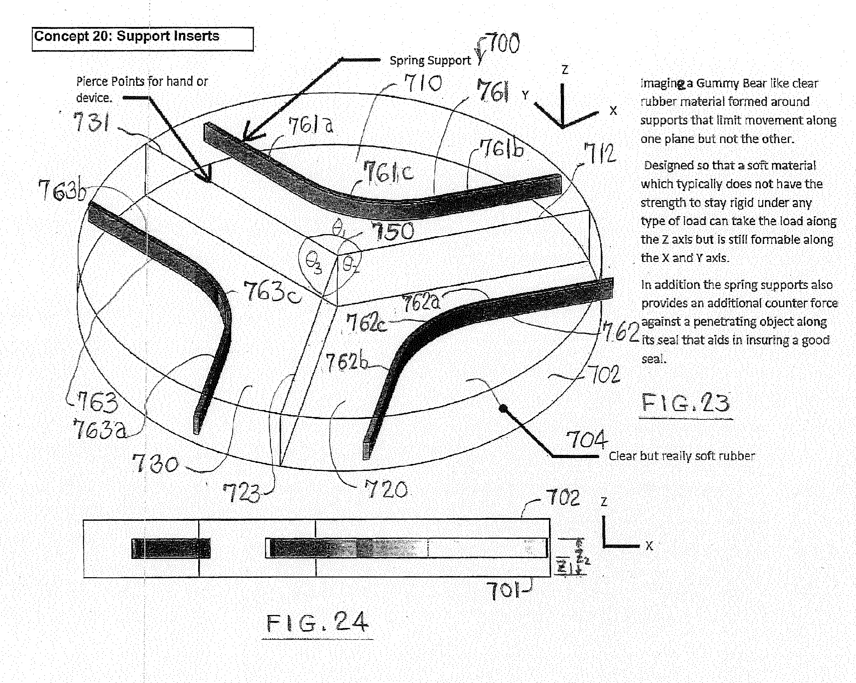

Turning now to FIGS. 23-27, FIG. 23 illustrates an instrument or hand access surgical site seal cap 700 having a series of flexible arcuate spring supports positioned within a soft rubber-like material such that the supports limit horizontal movement of the material but allow vertical movement according to one exemplary embodiment of the present disclosure.

More particularly, surgical site seal cap 700 includes a cylindrical retaining housing 702 which encases a flexible correspondingly cylindrically-shaped material 704 that is divided into a plurality of segments. The cylindrical retaining housing 702 extends vertically in the z-direction as defined by the x-y-z coordinates in FIG. 23. The x and y coordinates define a horizontal plane.

In the exemplary embodiment of FIGS. 23-27, the flexible material 704 is divided into three equal segments, i.e., first segment 710, second segment 720 and third segment 730. As a result of the division of the flexible material 704 into equal segments, a first access pierce slit 712 is formed at the interface between first segment 710 and second segment 720. A second access pierce slit 723 is formed at the interface between second segment 720 and third segment 730. Similarly, third access pierce slit 731 is formed at the interface between third segment 730 and first segment 710.

Each segment 710, 720, 730 spans an angle θ1, θ2, θ3, respectively, defined between the respective access pierce slits 712, 723, 731, that is equal to 120° having an origin or vertex 750 at the common intersection of the pierce slits 712, 723, 731.

The segments 710, 720, 730 each further include a respective bow-shaped or arcuate spring support 761, 762, 763 formed within the flexible material of the respective segment 710, 720, 730.

The first bow-shaped support 761 defines a first arm 761a and a second arm 761b extending from a common base 761c. The arms 761a and 761b straddle the pierce slits 712 and 731, respectively, while the base 761c interfaces the common origin 750.

Similarly, the second bow-shaped support 762 defines a first arm 762a and a second arm 762b extending from a common base 762c. The arms 762a and 762b straddle the pierce slits 712 and 723, respectively, while the base 762c interfaces the common origin 750.

Further, the third bow-shaped support 763 defines a first arm 763a and a second arm 763b extending from a common base 763c. The arms 763a and 763b straddle the pierce slits 723 and 731, respectively, while the base 763c interfaces the common origin 750.

Again, the soft rubber-like material 704 may be formed of a medical grade plastic or medical grade foam which may or may not be encased in a silicone material as described above or formed of a medical balloon material, e.g., latex, silicone rubber, Mylar®, polyethylene, polyurethane, a flexible polymeric material, or a composite material, such as a polyethylene and nylon composite, or any other resilient material suitable for the intended purpose of permitting insertion and manipulation, e.g., off-axis manipulation, of a surgical instrument or clinician's hand.

FIG. 24 is an elevation cross-sectional view along the X-axis of the instrument or hand access surgical site seal cap 700 showing the flexible bow-shaped or arcuate spring supports 761 and 763 positioned within the soft rubber-like material 704 between lower surface 701 and upper surface 702. The lower edge of each spring support 761 and 763 is positioned at an elevation Z1 in the z-direction above the lower surface 701 while the upper edge of each spring support 761 and 763 is positioned at an elevation Z2 in the Z-direction above the lower surface 701. Spring support 762, not shown, is similarly positioned within the rubber-like material 704.

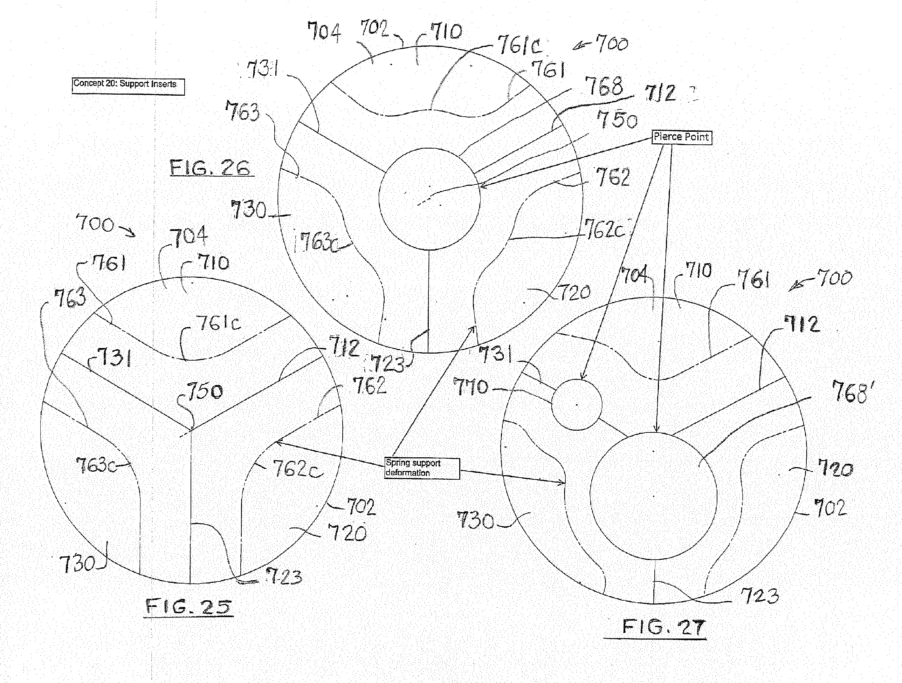

As a result of the location within the soft rubber-like material 704, the spring supports 761, 762, 763 limit movement in the Z-direction while allowing motion in the X and Y directions. The spring supports 761, 762, 763 also provide an additional counter force against a penetrating object along the seal or access pierce slits 712, 723, 731, as illustrated in FIGS. 26 and 27 and described below.

FIG 25 is a plan view of the instrument or hand access surgical site seal cap 700 showing the central intersection at origin or vertex 750 at the common intersection of the pierce slits 712, 723, 731 in the rubber-like material 704 that are positioned between the flexible arcuate spring supports 761, 762, 763 when there is neither an instrument or the hand of a surgeon positioned through the seal cap 700.

FIG. 26 is a plan view of the instrument or hand access surgical site seal cap 700 illustrating the deformation of the flexible arcuate spring supports 761, 762, 763 when a hand or an instrument 768 is disposed symmetrically through the central intersection 750 of the pierce slits 712, 723, 731. In this case, the common bases 761c, 762c, 763c are equally deflected away from the central intersection 750 in the horizontal x-y plane while maintaining a seal by the material 704 around the hand or instrument 768.

FIG. 27 is a plan view of the instrument or hand access surgical site seal cap 700 illustrating the deformation of the flexible arcuate spring supports 761, 762, 763 when a hand or an instrument 768' is disposed asymmetrically through the central intersection 750 of the pierce slits 712, 723, 731 and an instrument 770 having a small cross-sectional area is disposed asymmetrically through one of the pierce slits, e.g., pierce slit 731, in the rubber-like material 704. The flexible arcuate spring supports 761, 762, 763 distort in the horizontal x-y plane in a manner to mimic the circumference of the hand or instrument 768' and of the instrument 770.

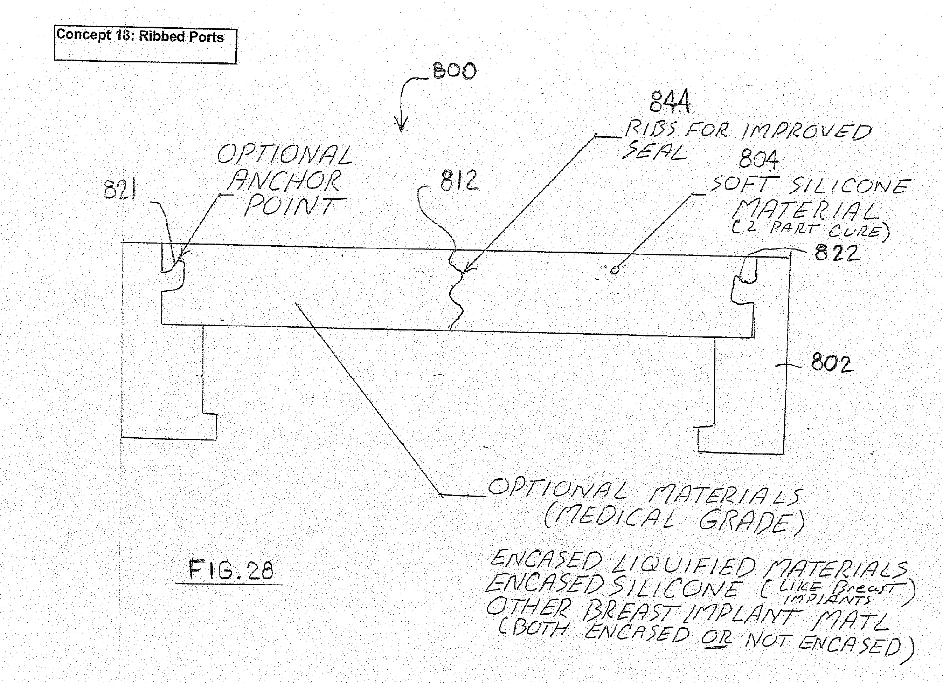

FIG. 28 is cross-sectional view of an alternate exemplary embodiment of the instrument or hand access surgical site seal cap 700 of FIGS. 23-27. More particularly, instrument or hand access surgical site seal cap 800 includes soft rubber-like material 804 mounted in a cylindrical housing 802.

The soft rubber-like material 804 includes pierce slits, e.g. pierce slit 812, that have a curled wave-like vertical profile to form interlocking ribs 844 to improve the seal capability.

The cylindrical housing 802 may further include one or more anchor points 821 and 822 disposed on opposite sides of the housing 802 to partially penetrate into the soft-rubber-like material 804. The anchor points 821 and 822 may have a J-shaped profile which is oriented to increase the vertical shear strength at the anchor points 821 and 822.

In a similar manner, the soft rubber-like material 804 may be formed of a medical grade plastic or medical grade foam which may or may not be encased in a silicone material as described above or formed of a medical balloon material, e.g., latex, silicone rubber, Mylar®, polyethylene, polyurethane, a flexible polymeric material, or a composite material, such as a polyethylene and nylon composite, or any other resilient material suitable for the intended purpose of permitting insertion and manipulation, e.g., off-axis manipulation, of a surgical instrument or clinician's hand.

The cylindrical housing 802 may be made from rigid members or alternatively made from a disposable, compressible, and/or flexible type material, for example, but not limited to, a suitable foam or gel material having sufficient compliance to form a seal about one or more surgical objects, shown generally as surgical object, and also establish a sealing relation with the tissue. The foam is preferably sufficiently compliant to accommodate off axis motion of the surgical object. In one embodiment, the foam may be at least partially constituted of polyisoprene, urethane, or silicone, or the like. Alternatively, cylindrical housing 802 may be formed of a biocompatible gel material. Again, suitable portal members are disclosed in commonly assigned U.S. patent application Ser. No. 12/244,024, filed Oct. 2, 2008, now abandoned, published as U.S. Patent Application Publication No. 2009/0093752 A1, the entire contents of which has already been hereby incorporated by reference above .

The foregoing materials may be applied to each of the embodiments of the instrument or hand access surgical site access caps 100 to 800 described above.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as examples of particular embodiments. Those skilled in the art will envision other modifications within the scope and spirit of the claims appended hereto.

Persons skilled in the art will understand that the various apparatus, and corresponding methods of use described herein, and shown in the accompanying drawings, constitute non-limiting, exemplary embodiments of the present disclosure, and that additional components and features may be added to any of the embodiments discussed herein above without departing from the scope of the present disclosure.

Additionally, persons skilled in the art will understand that the elements and features shown or described in connection with one exemplary embodiment may be combined with those of another embodiment without departing from the scope of the present disclosure, and will appreciate further features and advantages of the presently disclosed subject matter based on the above-described embodiments and the claims. Accordingly, the present disclosure is not limited by what has been particularly shown and described.

Although the foregoing disclosure has been described in some detail by way of illustration and example, for purposes of clarity or understanding, it will be obvious that certain changes and modifications may be practiced within the scope of the appended claims.

The invention may be described by reference to the following numbered paragraphs:-
1. A surgical access device comprising: a foam structure defining a plurality of generally triangular wedges circumferentially arranged about a longitudinal axis so as to seal the surgical incision, the plurality of generally triangular wedges defining, adjacent their respective apices, a central opening for sealed reception of a surgical object, and a second opening defined between a pair of adjacent wedges, the second opening configured to receive and seal with a surgical instrument inserted into the second opening.
2. The surgical access device of paragraph 1, wherein each wedge has a first vertical surface on a first side and a second vertical surface on a second side.
3. The surgical access device of paragraph 2, wherein a first vertical surface of a first wedge abuts a second vertical surface of a second wedge.
4. The surgical access device of paragraph 3, wherein the first vertical surface of the first wedge and the second vertical surface of the second wedge form a slit between the first and second wedges.
5. The surgical access device of paragraph 4, wherein the slit formed between the first vertical surface of the first wedge and the second vertical surface of the second wedge extends fully between an upper surface and a lower surface of the surgical access device.
6. The surgical access device of paragraph 1, wherein the second opening is in communication with at least a part of the slit.

## Claims

1. A surgical access device comprising:
a foam structure defining a plurality of generally triangular wedges circumferentially arranged about a longitudinal axis so as to seal the surgical incision, the plurality of generally triangular wedges defining, adjacent their respective apices, a central opening for sealed reception of a surgical object, and
a second opening defined between a pair of adjacent wedges, the second opening configured to receive and seal with a surgical instrument inserted into the second opening.

2. The surgical access device of claim 1, wherein each wedge has a first vertical surface on a first side and a second vertical surface on a second side.

3. The surgical access device of claim 2, wherein a first vertical surface of a first wedge abuts a second vertical surface of a second wedge.

4. The surgical access device of claim 3, wherein the first vertical surface of the first wedge and the second vertical surface of the second wedge form a slit between the first and second wedges.

5. The surgical access device of claim 4, wherein the slit formed between the first vertical surface of the first wedge and the second vertical surface of the second wedge extends fully between an upper surface and a lower surface of the surgical access device.

6. The surgical access device of claim 4 or claim 5, wherein the second opening is in communication with at least a part of the slit.
